# EUROPEAN PATENT SPECIFICATION

(11) **EP 1 935 614 B1**
(45) Date of publication and mention of the grant of the patent: **02.09.2009**
(21) Application number: 07254890.2
(22) Date of filing: 17.12.2007
(51) Int. Cl.: B29C 57/00, A61M 25/00

(54) **Apparatus and methods of end forming tubes**
Vorrichtung und Verfahren für endenformende Rohrleitungen
Appareil et procédés de formation d'extrémité de tubes

(30) Priority: 22.12.2006 GB 0625755
(43) Date of publication of application: 25.06.2008
(73) Proprietor: Smiths Group plc, London SW1E 5JL (GB)
(72) Inventor: Thomas, Karl Jolyon, Swindon Wiltshire SN25 4TP (GB)
(74) Representative: Flint, Jonathan McNeill

(56) References cited:
- EP-A- 0 847 771
- WO-A-03/043547

## Description

This invention relates to apparatus of the kind for end forming a tube including a mould defining a cavity with a smoothly-rounded closed end surface.

The invention is more particularly, but not exclusively concerned with apparatus and methods for end forming small diameter medical tubes and catheters, as for instance known from WO-A-03/043 547.

It is important for many medical tubes to have a smooth patient end in order to reduce trauma while in the patient. End forming such tubes does not generally cause any difficulty where the tubes are fairly large in diameter, typically over about 5mm in outside diameter. It is considerably more difficult, however, with open-ended smaller diameter tubes, since it is difficult to hold the end open during any end-forming operation. The finish of the end of many catheters having a diameter less than about 2mm is generally quite poor. This can be a particular problem in catheters used during IVF procedures, such as embryo transfer catheters. Such catheters have a very small diameter and the open patient end tip needs to be very smooth in order to prevent damage to the embryo. These catheters are usually used under microscope observation so imperfections in the catheter tip are readily apparent.

It is an object of the present invention to provide alternative apparatus and methods of end forming a tube.

According to one aspect of the present invention there is provided apparatus of the above-specified kind, characterised in that the apparatus includes a pin member arranged to be received within one end of the tube, a grip mechanism for gripping the tube and pin member with the pin member protruding from the end of the tube, an arrangement for heating the cavity, and an arrangement for advancing the assembly of the tube and pin member forwardly into the cavity such that the pin member initially contacts the closed surface of the cavity and the tube is then pushed forwardly relative to the pin member until the forward end of the tube contacts the closed end of the cavity and the heat of the cavity causes the material at the forward end of the tube to flow around the pin member and take up the shape of the cavity with the pin member keeping the forward end of the tube open.

The mould member is preferably of a thermally-insulative member such as glass and the pin member is preferably of a metal and may be hollow. The tube preferably has an external diameter of about 1mm. The apparatus may include an arrangement for cooling the outside of the mould after the material of the tube has flowed. The arrangement for heating the cavity preferably includes a heater in thermal contact with the outside of only the closed end of the mould.

According to another aspect of the present invention there is provided a method of end forming an open end of a plastics tube, including the steps of inserting a pin member into the forward end of the tube to be formed, gripping the tube and pin member through the tube member with the pin member protruding from the forward end of the tube, inserting the assembly of the pin member and tube into the open end of a heated cavity having a smoothly-rounded closed end, contacting the closed end of the cavity with the pin member and continuing to advance the assembly so that the tube is pushed forwardly relative to the pin member until the forward end of the tube contacts the closed end of the cavity and the material of the tube at its forward end is caused to flow to take up the smoothly rounded shape of the cavity while the pin member maintains the end of the tube open, subsequently withdrawing the assembly from the cavity and removing the pin member from the tube.

Preferably, the method includes the step of blowing cooling air onto the outside of the cavity after the material of the tube has flowed so that the flowed material starts to harden before the assembly is removed from the cavity.

Apparatus and a method for end forming tubes according to the present invention will now be described, by way of example, with reference to the accompanying drawing in which:
- Figure 1: is a side elevation view of the apparatus;
- Figure 2: is a cross-sectional side elevation of a part of the apparatus to a larger scale at a preliminary stage of insertion of the tube into the cavity;
- Figure 3: is a cross-sectional side elevation of the part shown in Figure 2 at a later stage when the tube has been fully inserted into the cavity; and
- Figure 4: is a plan view of a part of the apparatus to a larger scale.

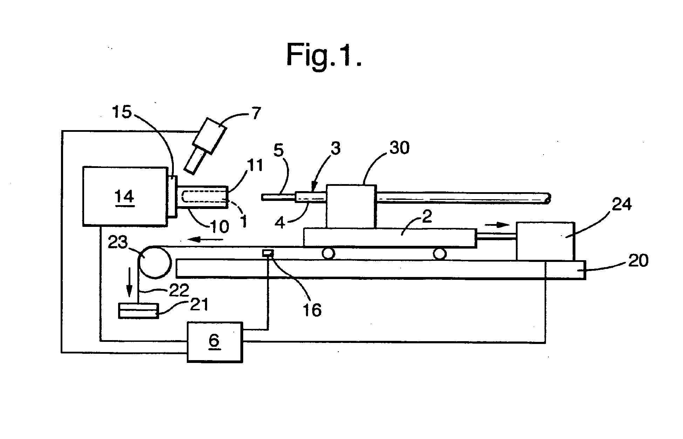

With reference first to Figure 1, the apparatus includes a fixed, heated mould cavity 1 arranged horizontally and a carriage 2 movable horizontally and supporting an assembly 3 of a tube 4 and pin 5 so that the assembly can be moved into the cavity and withdrawn out of the cavity when the end-forming operation has been completed.

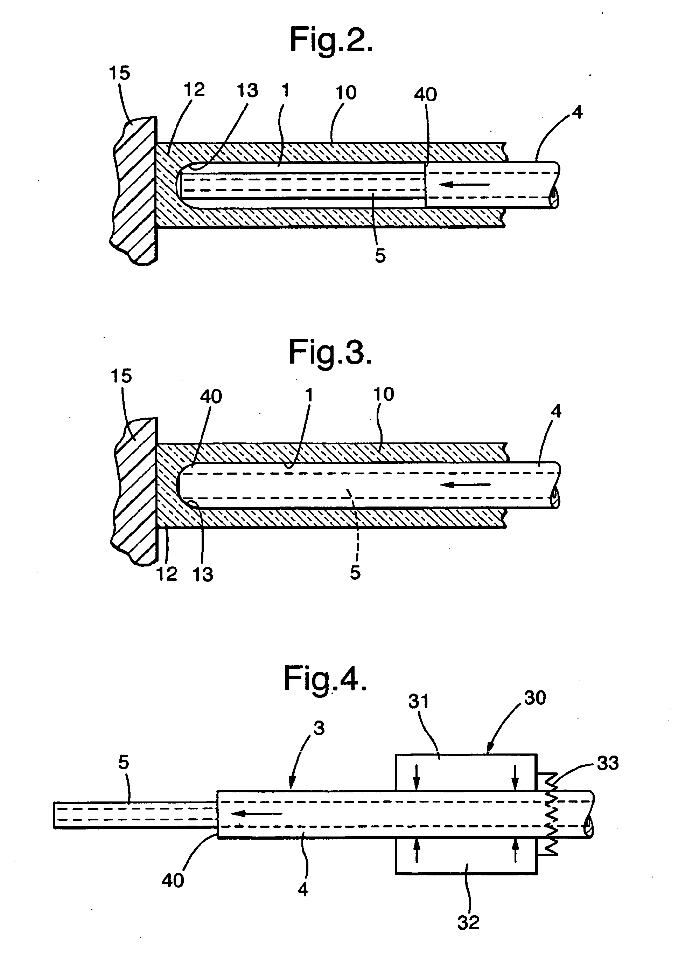

With reference now to Figures 2 and 3, the cavity 1 is provided by the bore of a mould member in the form of a rigid sleeve 10 of a heat-resistant glass material about 30mm long. The sleeve 10 is open at its right-hand end 11 and is closed at its left-hand end 12 by melting the end, twisting it closed and then re-heating and blowing the sleeve while hot to form a perfectly smooth rounded inner surface 13 of hemispherical shape, like a half bubble. The left-hand end 12 of the sleeve 10 is heated by an electrical resistance heater block 14 with temperature feedback control. The heater block 14 is selected to have a relatively large thermal mass so that its temperature can be maintained with a high degree of accuracy via a control unit 6. Heat is transferred from the heater block 14 to the sleeve 10 by means of a high-temperature silicone rubber pad 15 on the right-hand end of the block and abutted by the left-hand, closed end 12 of the glass sleeve. The resilience of the pad 15 accommodates irregularities in the end of the mould sleeve 10 and thereby ensures effective thermal contact. The relatively poor thermal conductivity of the glass material of the sleeve 10 means that the closed end 12 of the cavity 1 can be heated with little heating effect on the remainder of the sleeve and cavity.

Alongside the closed end 12 of the mould sleeve 10 is located cooling means in the form of a cold air blower 7 arranged to direct a cooling jet of air onto the external surface of the left-hand end of the sleeve when energised by the control unit 6.

The carriage 2 is slidable relative to a fixed base 20 backwards and forwards along a horizontal path aligned axially of the mould sleeve 10. The carriage 2 is urged forwardly by means of a weight 21 attached to the left-hand end of the carriage by a cable 22. The cable 22 passes horizontally from the carriage 2, over a pulley wheel 23 and hangs vertically down suspending the weight so that it applies a force to move the carriage to the left. A pneumatic actuator 24, or other linear actuator, is mounted on the base 20 and is coupled to the rear, right-hand end of the carriage 2. The actuator 24 is controlled by the control unit 6 and is operable to allow free movement of the carriage 2 to the left caused by the suspended weight 21, but, when energised pulls the carriage to the right, against the action of the weight. The carriage 2 supports a grip mechanism 30 comprising two gripper arms 31 and 32 urged together in a horizontal direction transverse to the axis of the mould sleeve 10 with a controlled force by means of a spring 33, as shown in Figure 4. The arms 31 and 32 act to grip the assembly 3 of the tube 4 and the pin 5.

The tube 4 is of a polyurethane, or some other thermally-formable plastics material, and is intended to be formed into an embryo transfer catheter. Typically, the outside diameter of the tube 4 would be about 1mm, the internal diameter of the mould sleeve 10 being selected so that the tube is a close sliding fit within the mould sleeve. The right-hand machine end of the tube 4 is not shown and could be plain, for subsequent attachment to a suitable hub, or it could be already attached to a hub. The left-hand, patient end 40 of the tube 4 is cut square and has initially a relatively sharp edge. The pin 5 is hollow, about 50mm long with a circular external shape and a diameter that enables it to slide into the patient end of the tube 4 as a close fit. The pin 5 is made of a metal, such as stainless steel, or some other stiff and preferably thermally-conductive material.

The manufacturer initially takes the tube 4 and threads one end of the metal pin 5 into its forward, patient end to leave about 5mm projecting. He then takes the assembly 3 and loads it onto the carriage 2 (the carriage being held in a position towards the right hand end of the base 2 with the weight in an elevated position) by opening the gripper arms 31 and 32. He then releases the arms 31 and 32 onto the assembly 3, in the manner shown in Figure 4, so that they grip both the outside of the tube 4 and the pin 5 by squeezing the inside of the tube against the outside of the pin. The length of the pin 5 is not important but it should be sufficient to extend to the right of the gripper arms 31 and 32 while projecting out of the left-hand end of the tube 4. The resilient force applied by this grip is carefully selected such that it holds the pin 5 in position but allows it to be pushed rearwardly into the tube 4 in the manner described later. The positioning of the gripper arms 31 and 32 is such that the assembly 3 is aligned vertically and horizontally with the axis of the mould sleeve 10.

The carriage 2 is then released so that the weight 21 moves it with a controlled force to the left. The assembly 3 thereby enters the cavity 1 until the position shown in Figure 2 is reached where the tip of the pin 5 contacts the closed end 13 of the cavity. The force applied to move the carriage 2 to the left and the force with which the pin 5 is gripped by the tube 4 is selected to be such that the carriage continues to be displaced to the left causing the tube to slide forwardly in the cavity 1 along the outside of the pin, which remains firmly contacting the floor 13 of the cavity. The hollow nature of the pin 5 allows air trapped in the cavity 1 to escape as the tube 4 is advanced, via the bore of the pin and along the bore of the tube. The heat applied by the heater block 14 via the pad 15 is selected to raise the temperature inside the left-hand end of the cavity 1 to within the plastic processing range of the tube material, in which it will flow without becoming too liquid. When the tip 40 of the tube 4 reaches this quite localized heated region of the cavity 1 its material softens and flows around the side of the pin 5 to conform to the external curved surface of the pin 5 and the surface 13 of the cavity 1. At this point in the travel of the carriage 2 it is arranged to trip a microswitch 16 or other position sensor, which provides a signal to the control 6 to start a timer. After a set time sufficient for material at the tip 40 of the tube 4 to have flowed sufficiently, the control unit 6 turns on the cold air blower 7 so as to cool the outside and hence also the inside of the mould sleeve 10. This causes a drop in temperature within the cavity 1 and the plastic material of the tube 4 to start to harden. This cooling step has been found useful to reduce the amount of plastic remaining in the cavity 1 after withdrawal of the tube 4. After a second predetermined time, the control unit 6 sends a signal to the actuator 24 to pull the carriage 2 to the right and withdraw the assembly 3 from the cavity 1. At the same time, the control unit 6 stops the blower 7 to allow the cavity 1 to heat up again for the next end forming operation. The user then unclips the assembly 3 from the gripper arms 31 and 32 and removes the metal pin 5, which can be achieved easily by bending and manipulating the tube 4 at the right-hand end of the pin so as to push it out of the formed, left-hand, patient end of the tube. After removal of the pin 5 the patient end 40 of the tube 4 can be seen to have been formed with a smoothly-rounded end tip.

The apparatus and method enables consistent, high quality end forming of plastic tubes. It lends itself to automation although could also be carried out manually by gripping and inserting the assembly into the heated cavity by hand. When automated, equipment may be provided with multiple stations on a rotating head so that tubes are inserted into and removed from the heated cavity each time the head is rotated by one station.

The invention is not confined to medical tubes but could be used to end form other plastic tubes and is especially suitable for those of relatively small diameter. The mould need not be made of glass although this has been found to enable a very smooth finish. Alternative, preferably thermally-insulative materials could be used. The pin need not be of a metal but it should be stiff and relatively robust. It has also been found preferable for a thermally-conductive material to be used to help dissipate the heat of the cavity and promote cooling of the flowed tube material. Clearly, the pin should not be of a material that will bond to the flowed tube material. Other arrangements could be used to displace the assembly of the tube and pin instead of the weight arrangement described. It is not essential that the assembly be moved horizontally since other orientations, such as a vertical orientation and displacement are possible.

## Claims

1. Apparatus for end forming a tube (4) including a mould (10) defining a cavity (1) with a smoothly-rounded closed end surface (13), **characterised in that** the apparatus includes a pin member (5) arranged to be received within one end (40) of the tube (4), a grip mechanism (30) for gripping the tube (4) and pin member (5) with the pin member protruding from the end (40) of the tube, an arrangement (14, 15) for heating the cavity (1), and an arrangement (2, 21, 22, 23) for advancing the assembly (3) of the tube (4) and pin member (5) forwardly into the cavity (1) such that the pin member (5) initially contacts the closed surface (13) of the cavity (1) and the tube (4) is then pushed forwardly relative to the pin member (5) until the forward end (40) of the tube (4) contacts the closed end (13) of the cavity (1) and the heat of the cavity causes the material at the forward end (40) of the tube (4) to flow around the pin member (5) and take up the shape of the cavity (1) with the pin member (5) keeping the forward end (40) of the tube (4) open.

2. Apparatus according to Claim 1, **characterised in that** the mould (10) is of a thermally-insulative material.

3. Apparatus according to Claim 2, **characterised in that** the mould (10) is of glass.

4. Apparatus according to any one of the preceding claims, **characterised in that** pin member (5) is of metal.

5. Apparatus according to any one of the preceding claims, **characterised in that** the pin member (5) is hollow.

6. Apparatus according to any one of the preceding claims, **characterised in that** the tube (4) has an external diameter of about 1mm.

7. Apparatus according to any one of the preceding claims, **characterised in that** the apparatus includes an arrangement (7) for cooling the outside of the mould (10) after the material of the tube (4) has flowed.

8. Apparatus according to any one of the preceding claims, **characterised in that** the arrangement for heating the cavity (1) includes a heater (14) in thermal contact with the outside of only the closed end (12) of the mould (10).

9. A method of end forming an open end of a plastics tube (4), including the steps of inserting a pin member (5) into the forward end (40) of the tube to be formed, gripping the tube (4) and pin member (5) through the tube member with the pin member protruding from the forward end (40) of the tube, inserting the assembly (3) of the pin member (5) and tube (4) into the open end of a heated cavity (1) having a smoothly-rounded closed end (13), contacting the closed end (13) of the cavity (1) with the pin member (5) and continuing to advance the assembly (3) so that the tube (4) is pushed forwardly relative to the pin member (5) until the forward end (40) of the tube (4) contacts the closed end (13) of the cavity (1) and the material of the tube at its forward end (40) is caused to flow to take up the smoothly rounded shape of the cavity (1) while the pin member (5) maintains the end (40) of the tube open, subsequently withdrawing the assembly (3) from the cavity (1) and removing the pin member (5) from the tube (4).

10. A method according to Claim 9, **characterised in that** the method includes the step of blowing cooling air onto the outside of the cavity (10) after the material of the tube (4) has flowed so that the flowed material starts to harden before the assembly (3) is removed from the cavity (1).

## Patentansprüche

1. Gerät zur Endformung eines Tubus (4), umfassend eine Hohlform (10), welche einen Hohlraum (1) mit einer sanft abgerundeten geschlossenen Endoberfläche (13) definiert, **dadurch gekennzeichnet, dass** der Apparat ein zur Aufnahme an einem Ende (40) des Tubus (4) ausgebildetes Stiftteil (5), einen Greifmechanismus (30) zum Greifen des Tubus (4) und des Stiftteils (5) mit dem vom Ende (40) des Tubus hervorstehenden Stiftteil, eine Anordnung (14, 15) zum Heizen des Hohlraums (1), und eine Anordnung (2, 21, 22, 23) zum Vorschieben der aus Tubus (4) und Stiftteil (5) gebildeten Einheit (3) in den Hohlraum (1), so dass das Stiftteil (5) zuerst die geschlossene Oberfläche (13) des Hohlraums (1) berührt und der Tubus (4) dann relativ zum Stiftteil (5) vorwärts gedrückt wird, bis das vordere Ende (40) des Tubus (4) das geschlossene Ende (13) des Hohlraums (1) berührt und die Hitze des Hohlraums das Material am vorderen Ende (40) des Tubus (4) dazu bringt, um das Stiftteil (5) zu fließen und die Form des Hohlraums (1) anzunehmen, wobei das Stiftteil (5) das vordere Ende (40) des Tubus (4) offenhält, umfasst.

2. Gerät nach Anspruch 1, **dadurch gekennzeichnet, dass** die Hohlform (10) aus einem thermisch isolierenden Material besteht.

3. Gerät nach Anspruch 2, **dadurch gekennzeichnet, dass** die Hohlform (10) aus Glas besteht.

4. Gerät nach einem der voranstehenden Ansprüche, **dadurch gekennzeichnet, dass** das Stiftteil (5) aus Metall besteht.

5. Gerät nach einem der voranstehenden Ansprüche, **dadurch gekennzeichnet, dass** das Stiftteil (5) hohl ist.

6. Gerät nach einem der voranstehenden Ansprüche, **dadurch gekennzeichnet, dass** der Tubus (4) einen Außendurchmesser von etwa 1 mm hat.

7. Gerät nach einem der voranstehenden Ansprüche, **dadurch gekennzeichnet, dass** der Apparat eine Anordnung (7) zum Kühlen der Außenseite der Hohlform (10), nachdem das Material des Tubus (4) geflossen ist, hat.

8. Gerät nach einem der voranstehenden Ansprüche, **dadurch gekennzeichnet, dass** die Anordnung zum Heizen der Hohlraum (1) eine in thermischen Kontakt mit der Außenseite nur des geschlossenen Endes (12) der Hohlform (10) stehende Heizung (14) enthält.

9. Verfahren zum Endformen eines offenen Endes eines Plastiktubus (4), umfassend die Schritte des Einsetzens eines Stiftteils (5) in das vordere Ende (4) des zu formenden Tubus, Greifen des Tubus (4) und des Stiftteils (5) durch das Tubusteil mit dem vom vorderen Ende (40) des Tubus hervorstehenden Stiftteil, Einsetzen der aus dem Stiftteil (5) und dem Tubus (4) gebildeten Einheit (3) in das offene Ende eines mit einem sanft abgerundeten geschlossenen Ende (13) versehenen geheizten Hohlraums (1), Kontaktieren des geschlossenen Endes (13) des Hohlraums (1) mit dem Stiftteil (5) und stetiger Vorschub der Einheit (3), so dass der Tubus (4) relativ zum Stiftteil (5) vorwärts geschoben wird, bis das vordere Ende (40) des Tubus (4) das geschlossene Ende (13) des Hohlraums (1) berührt und das Material des Tubus an seinem vorderen Ende (40) zum Fließen gebracht wird, um die sanft abgerundete Form des Hohlraums (1) anzunehmen, während das Stiftteil (5) das Ende (40) des Tubus offenhält, und nachfolgendes Entnehmen der Einheit (3) aus dem Hohlraum (1) und Entfernen des Stiftteils (5) aus dem Tubus (4).

10. Verfahren nach Anspruch 9, **gekennzeichnet durch** den Schritt des Blasens von Kühlluft auf die Außenseite des Hohlraums (1), nachdem das Material des Tubus (4) geflossen ist, so dass das geflossene Material auszuhärten beginnt, bevor die Einheit (3) vom Hohlraum (1) entfernt wird.

## Revendications

1. Appareil pour le façonnage de l'extrémité d'un tube (4), comprenant un moule (10) qui définit une cavité (1) possédant une surface d'extrémité fermée (13) largement arrondie, **caractérisé en ce que** l'appareil comprend un élément formant broche (5), agencé pour être reçu dans une extrémité (40) du tube (4), un mécanisme de préhension (30), destiné à saisir le tube (4) et l'élément formant broche (5), l'élément formant broche faisant saillie au-delà de l'extrémité (40) du tube, un dispositif (14, 15) destiné à chauffer la cavité (1) et un dispositif (2, 21, 22, 23) destiné à faire avancer l'ensemble (3) du tube (4) et de l'élément formant broche (5) vers l'avant en l'enfonçant dans la cavité (1) de telle manière que l'élément formant broche (5) entre initialement en contact avec la surface fermée (13) de la cavité (1), et le tube (4) est ensuite poussé vers l'avant par rapport à l'élément formant broche (5) jusqu'à ce que l'extrémité avant (40) du tube (4) entre en contact avec l'extrémité fermée (13) de la cavité (1) et que la chaleur de la cavité amène :a matière de l'extrémité avant (40) du tube (4) à fluer autour de l'élément formant broche (5) et à prendre la forme de la cavité (1), l'élément formant broche (5) maintenant alors l'extrémité avant (40) du tube (4) ouverte.

2. Appareil selon la revendication 1, **caractérisé en ce que** le moule (10) est réalisé en une matière thermiquement isolante.

3. Appareil selon la revendication 2, **caractérisé en ce que** le moule (10) est réalisé en verre.

4. Appareil selon l'une quelconque des revendications précédentes, **caractérisé en ce que** l'élément formant broche (5) est réalisé en métal.

5. Appareil selon l'une quelconque des revendications précédentes, **caractérisé en ce que** l'élément formant broche (5) est creux.

6. Appareil selon l'une quelconque des revendications précédentes, **caractérisé en ce que** le tube (4) comporte un diamètre extérieur d'environ 1 mm.

7. Appareil selon l'une quelconque des revendications précédentes, **caractérisé en ce que** l'appareil comprend un dispositif (7) destiné à refroidir la surface externe du moule (10) lorsque la matière du tube (4) a flué.

8. Appareil selon l'une quelconque des revendications précédentes, **caractérisé en ce que** le dispositif destiné à chauffer la cavité (1) comprend un élément chauffant (14) qui est en contact thermique avec la surface externe de la seule extrémité fermée (12) du moule (10).

9. Procédé pour façonner l'extrémité ouverte d'un tube en matière plastique (4), comprenant les étapes consistant à insérer un élément formant broche (5) dans l'extrémité avant (40) du tube à façonner, à saisir le tube (4) et l'élément formant broche (5) à travers l'élément formant tube l'élément formant broche débordant au-delà de l'extrémité avant (40) du tube, à insérer l'ensemble (3) de l'élément formant broche (5) et du tube (4) dans l'extrémité ouverte d'une cavité chauffée (1) ayant une extrémité fermée largement arrondie (13), à mettre en contact l'extrémité fermée (13) de la cavité (1) avec l'élément formant broche (5) et à continuer à faire avancer l'ensemble (3) de manière que le tube (4) soit poussé vers l'avant par rapport à l'élément formant broche (5) jusqu'à ce que l'extrémité avant (40) du tube (4) entre en contact avec l'extrémité fermée (13) de la cavité (1) et que la matière du tube, à son extrémité avant (40), soit amenée à fluer pour prendre la forme largement arrondie de la cavité (1) pendant que l'élément formant broche (5) maintient l'extrémité (40) du tube ouverte, à extraire par la suite l'ensemble (3) de la cavité (1) et à retirer l'élément formant broche (5) du tube (4).

10. Procédé selon la revendication 9, **caractérisé en ce que** le procédé comprend l'étape consistant à souffler de l'air de refroidissement sur la surface externe de la cavité (10) lorsque la matière du tube (4) a flué, de façon que la matière fluée commence à durcir avant que l'ensemble (3) ait été retiré de la cavité (1).
